# EUROPEAN PATENT APPLICATION

(11) **EP 4 190 444 A1**
(43) Date of publication of application: **07.06.2023**
(21) Application number: 21850023.9
(22) Date of filing: 27.07.2021
(51) Int. Cl.: B01J 23/06, B01J 23/08, B01J 29/40, C07C 1/20, C07C 15/08

(54) **CORE-SHELL COMPOSITE CATALYST, PREPARATION METHOD FOR SAME, AND USE THEREOF**

(30) Priority: 30.07.2020 CN 202010752882
(71) Applicant: HighChem Co., Ltd, Tokyo 105--0001 (JP); Mohan Co. Ltd, Toyama City 930-0875 (JP)
(72) Inventor: TSUBAKI, Noritatsu, Toyama City 930-0875 (JP); YANG, Guohui, Toyama City 930-0875 (JP); TAKA, Ushio, Beijing 100005 (CN)
(74) Representative: karo IP
(86) International application number: PCT/CN2021/108678
(87) International publication number: WO 2022/022510

(57) **Abstract**

The present invention relates to a core-shell composite catalyst, wherein the core is a spinel-structure XYₐO_{b} catalyst, wherein X and Y, being different from each other, are metal elements selected from main group II, transition elements and main group III of the Periodic Table of Elements; a is a number between 1-15, preferably between 1-5; b is the number of oxygen atoms required to satisfy the valence of the elements; the shell is a molecular sieve catalyst, preferably selected from one or more of ZSM-5, ZSM-11, ZSM-35 and MOR, more preferably selected from ZSM-5 and ZSM-11. When the core-shell composite catalyst is used for preparing p-xylene directly from syngas in one step, the process is simple and easy to operate; the selectivity toward p-xylene in xylene products is high; the conversion of syngas is high; and the service life of the catalyst is long. In addition, the present invention also relates to the preparation method of core-shell composite catalyst, and use thereof as the catalyst in the one-step preparation of p-xylene from syngas.

## Description

### Technical Field

The present invention relates to a core-shell composite catalyst, preparation method thereof, and use thereof in a one-step preparation of p-xylene from syngas.

### Background Art

Xylene is an important organic chemical material, which is widely used in packaging, fiber and other fields. P-xylene is mainly used to manufacture terephthalic acid, which can be used in the pharmaceutical industry. In addition, p-xylene is also an important intermediate used in the production of polyester fibers and industrial plastics. At present, industrial production methods of p-xylene mainly include toluene disproportionation and C₉ aromatic hydrocarbons transalkylation, xylene isomerization, xylene adsorptive separation and other technologies. In the disproportionation and transalkylation route, thermodynamics limits the content of p-xylene in the product, and only about 24 wt% of p-xylene content can be collected. The production of polyester fibers requires the concentration of p-xylene to be above 60 wt%, so the concentration of p-xylene obtained by current means is far from meeting the requirements of industrial production. In order to improve the concentration and yield of p-xylene, a series of workups are required. The physical properties of the three isomers of xylene, especially the small differences in boiling point, bring difficulties to the separation of p-xylene in C₈ aromatics. Therefore, it is necessary to adopt expensive adsorptive separation processes, which lead to the consumption of raw materials and rising costs. Currently, above 90% of aromatics come from the petroleum industry. However, in recent years, petroleum energy has become increasingly scarce. It is of great value to develop new processes and routes for aromatic synthesis, whether from the perspective of market demand or from the perspective of alternative energy.

As a bridge for energy conversion, syngas comes from natural resources such as natural gas, coal, biomass, and the like, and also can be converted into clean oil products with high added values, so it is considered as one of the most potential alternatives to petroleum. In recent years, a variety of new catalysts, led by the composite catalysts of metal catalysts and appropriate molecular sieves, have successfully modulated the distribution of Fischer-Tropsch reaction products and made a significant progress. After successfully using Fischer-Tropsch synthesis to produce oil products with different carbon number ranges, more and more researchers focus on processes of converting syngas into high value-added chemicals in one step with high selectivity. The high value-added chemicals include important chemical feedstock such as low carbon olefins and low carbon alcohols. Although methods related to the one-step synthesis of benzene, toluene, xylene and other aromatics from syngas have been reported constantly, the selectivity toward p-xylene in the products is usually not high and there is still a considerable distance from industrialization. The main problem is that it is difficult to make a breakthrough in the modulating of product selectivity, and another problem is that the catalyst is easy to be deactivated, which makes it difficult to maintain the stability of catalytic performance.

The key to improve the selectivity toward p-xylene prepared from syngas in one step is to develop high performance catalysts. It is found that composite bifunctional catalysts formed by combining a catalyst for synthesizing methanol or dimethyl ether with a molecular sieve, have good catalytic performance. This reaction route derives a series of cascade reactions: hydrogenation of syngas to methanol and dehydration of methanol to dimethyl ether, aromatization, xylene alkylation and isomerization, etc. This route is of great significance to the development of syngas conversion process. It not only fills the gap of relevant process routes, but also ensures the strategic security of national energy, and also provides a solution to the potential threat of world oil energy depletion. Therefore, further studying this route, such as improving the selectivity toward p-xylene and reducing the process complexity and cost, is an urgent technical difficulty to be solved in the one-step preparation of p-xylene from syngas.

In an earlier time, Lasa, et al. reported the performance of Cr-Zn/ZSM-5 catalysts for producing aromatics from syngas (Ind. Eng. Chem. Res., 1991, 30, 1448-1455), wherein among the hydrocarbon products, the selectivity toward aromatics was above 70%, but no mention was made of the specific product distribution of aromatics. Lately, Lasa, et al. reported using Cr-Zn/ZSM-5 composite catalysts to produce aromatics from syngas (Appl. Catalog. A, 1995, 125, 81-98), the selectivity toward aromatics in the hydrocarbon products was 75% under the reaction conditions of 356-410°C and 3.6-4.5 MPa, but the selectivity toward xylene did not exceed 20%. Guan Naijia, et al. (Catalog. Today, 1996, 30 (1-3), 207-213) from Nankai University reported that the CO conversion was 98.1% by using FeMnO-ZnZSM-5 composite catalysts under the reaction conditions of 270°C and 1.1 MPa, and the catalyst activity remained stable for 60 consecutive hours. The selectivity toward aromatics in the hydrocarbon products was up to 53.1 %, but no mention was made of the specific product distribution of xylene and benzene homologues with carbon number above 9. Recently, Ma Ding and Fan Weibin's team (Chem, 2017, 3, 323-333) coupled Na-Zn-Fe₅C₂ (FeZnNa) catalysts with mesoporous H-ZSM-5 molecular sieves with a multi-layer porous structure, and successfully converted syngas to aromatics via olefin intermediates in one step. Under the conditions of 340°C and 2 MPa, the conversion of CO was above 85%, and the catalytic system was stable and not easy to be deactivated. There were up to 51 wt% of aromatics in the hydrocarbon products, which predominantly consisted of light aromatics BTX (benzene, toluene, xylene). However, the selectivity toward p-xylene in the aromatics was not mentioned. The research group of Wang Ye (Chem, 2017, 3, 334-347) from Xiamen University successfully developed a hybrid catalyst of Zn-modified ZrO₂ and H-ZSM-5, with the CO conversion being 20% and the aromatic selectivity being 80%. In order to increase the content of light aromatics, they treated the outer surface of H-ZSM-5 with TEOS (ethyl orthosilicate) according to the chemical deposition method, which significantly improved the BTX selectivity in the final product, up to 60 wt%, but no mention was made of the content of xylene and p-xylene. In 2019, a relevant patent application (titled "Catalyst for directly preparing p-xylene from syngas, preparation thereof and applications thereof", Application Publication Number: CN109590019A) of Tsubaki Noritatsu (Toyama City, Japan), Yang Guohui, Gao Chao, Chai Jianyu, et al. discloses the preparation of p-xylene from syngas with high selectivity. Under the optimal reaction conditions, the conversion of carbon monoxide was 55%, and the selectivity toward p-xylene was 35.3%. Although the core-shell composite catalyst of this patent application reduces the surface acidity of the molecular sieve catalyst to some extent, the physical grinding, which is used to mix the molecular sieve catalyst and the metal oxide catalyst, makes a part of the intermediate product (methanol) cannot be immediately used for the next step reaction via the molecular sieve catalyst. Therefore, it is still necessary to develop more efficient catalysts.

Although the above catalysts all can convert syngas into aromatics in one step, either the selectivity toward p-xylene in the products or the conversion of carbon monoxide is not high, and the isomerization of xylene cannot be effectively controlled. Moreover, it is still a challenge to utilize the steric position of catalysts more efficiently.

### Contents of the Invention

The present invention aims to provide a new core-shell composite catalyst, preparation method thereof, and use thereof in the highly selective preparation of p-xylene from syngas via methanol. The catalyst is simple to prepare; the conversion of syngas is high; the selectivity toward p-xylene in xylene products is high; the service life is long; and the industrial application prospect is broad.

It is an object of the present invention to provide a core-shell composite catalyst. When the core-shell composite catalyst is used for the one-step conversion of syngas to p-xylene, the process is simple and easy to operate; the conversion of syngas is high; the selectivity toward p-xylene in xylenes is also high; and the service life of the core-shell composite catalyst is long.

Another object of the present invention is to provide a method for preparing the core-shell composite catalyst of the present invention.

A further object of the present invention is to provide use of the core-shell composite catalyst of the present invention or the core-shell composite catalyst obtained by the method of the present invention as the catalyst in the one-step preparation of p-xylene from syngas.

These and other purposes, features and advantages of the present invention will be easily understood by those skilled in the art by referring to the description below.

### Brief Description of the Figure

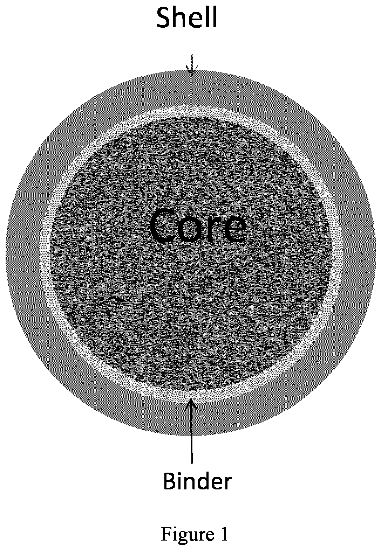

Figure 1 is a simplified structural diagram of the core-shell composite catalyst prepared according to Example 4, wherein the core is a spinel-structure catalyst, the shell is a molecular sieve catalyst, wherein there is a binder layer between them.

### Modes for Carrying out the Invention

According to the first aspect of the present invention, a core-shell composite catalyst is provided, wherein the core is a spinel-structure XYₐO_{b} catalyst, wherein X and Y, being different from each other, are metal elements selected from main group II, transition elements and main group III of the Periodic Table of Elements; a is a number between 1-15, preferably between 1-5; and b is the number of oxygen atoms required to satisfy the valence of the elements; wherein the shell is a molecular sieve catalyst, preferably selected from one or more of ZSM-5, ZSM-11, ZSM-35 and MOR, more preferably selected from ZSM-5 and ZSM-11.

In the core-shell composite catalyst of the present invention, the weight ratio of the spinel-structure XYₐO_{b} catalyst to the molecular sieve catalyst is 150:1-1:50, preferably 20:1-1:20, more preferably 10:1-1:10.

In the core-shell composite catalyst of the present invention, the core is a spinel-structure XYₐO_{b} catalyst, wherein X is a metal element selected from main group II, transition elements and main group III of the Periodic Table of Elements, preferably from Al, Ga, In, Tl, Zn, Cu, Co, Fe, Mn, Cr, Ti, Mg, Ca and Ba, more preferably from Ga, In and Zn; Y is a metal element selected from main group II, transition elements and main group III of the Periodic Table of Elements, preferably from Al, Ga, In, Tl, Zn, Cu, Co, Fe, Mn, Cr, Ti, Mg, Ca and Ba, more preferably from Cr, Ga and Ti. Preferably, the spinel-structure XYₐO_{b} catalyst is ZnCr₂O₄ or InGa₂O₄.

The spinel-structure XYₐO_{b} catalyst of the present invention can be prepared by any conventional methods in the art, such as sequential impregnation, co-impregnation and co-precipitation, preferably co-precipitation. When the above methods are used to prepare spinel-structure catalysts containing a first metal component X and a second metal component Y, they all involve mixing an aqueous metal salt solution containing the first metal component X and an aqueous metal salt solution containing the second metal component Y. After the catalyst is prepared, it is dried and calcined. The calcination atmosphere is air; the calcination temperature is 500-600°C, preferably 550-600°C; and the calcination time is 5-9 h, preferably 5-6 h.

For example, when the spinel-structure XYₐO_{b} catalyst is prepared by co-precipitation, aqueous solutions of soluble metal salts of X and Y are usually mixed, then dried and calcined. During the mixing process, a precipitant can be added to keep the pH value to be 8-9. The soluble salts are, for example, nitrates, chlorides, etc. The precipitant is, for example, an alkaline substance, such as NaOH, Na₂CO₃, NaHCO₃, (NH₄)₂CO₃, NH₄OH or ammonia-water. The mixing is carried out under heating and stirring. The heating temperature is 50-100°C, preferably 60-90°C, more preferably 70-80°C. The reaction time is 0.5-5 h, preferably 1-4 h. After the reaction, the reaction mixture is aged at the reaction temperature. The aging time is 0.5-10 h, preferably 1-5 h.

Preferably, in the co-precipitation method, a mixed solution a containing X and Y and a solution b containing the precipitant are added to a reactor by means of a pump, respectively. The addition rate of the two should ensure that the pH value of the mixture formed by the added solution a and the added solution b is 8-9 in real time.

For example, the process for preparing ZnCr₂O₄, a spinel-structure XYₐO_{b} catalyst, by co-precipitation is as follows: for preparing this catalyst, a mixed aqueous nitrate solution is formed by mixing respective nitrate salts of chromium and zinc with deionized water according to the required chromium/zinc ratio; and this solution is added dropwise together with an aqueous solution of ammonium carbonate (other precipitants, such as sodium carbonate, sodium hydroxide and ammonium hydroxide, may also be used) into a beaker with a pump for co-precipitation. During the co-precipitation process, the mixture is stirred continuously. The precipitation temperature is kept at 50-100°C, and the pH value is always controlled to be 8-9 by controlling the relative addition speeds of the two solutions. After the completion of the dropwise addition, stirring is stopped, and the mixture is kept at reaction temperature for 3-5 h for aging; the aged precipitates are filtered and washed with deionized water; the washed product is dried, and then calcined to obtain a ZnCr₂O₄ catalyst.

The shell of the core-shell composite catalyst of the present invention is a molecular sieve catalyst, preferably selected from one or more of ZSM-5, ZSM-11, ZSM-35 and MOR, more preferably selected from ZSM-5 and ZSM-11.

The molecular sieve catalyst of the present invention can be commercially obtained or prepared by any conventional methods in the art, such as hydrothermal synthesis, impregnation, ion exchanging, vapor phase deposition, liquid phase deposition, etc. Generally, the molecular sieve catalyst can be prepared by hydrothermal synthesis.

For example, the process for preparing an H-ZSM-5 zeolitic molecular sieve by hydrothermal synthesis is as follows: a mixture is formed from a silicon source (such as TEOS), an aluminum source (such as Al(NO₃)₃•9H₂O), an organic template (such as tetrapropylammonium hydroxide (TPAOH)), ethanol and deionized water, which is stirred for 2-10 h at room temperature to obtain a sol. Then the stirred sol is placed into a hydrothermal synthesis reactor, sealed, and crystallized for 12-72 h at a rotating speed of 2-5 rpm at a temperature of 160-200°C. After the completion of the crystallization, the product is cooled to room temperature and washed until the filtrate has a pH=7-8, dried overnight, and then heated in a muffle furnace to 400-650°C at a ramping rate of 0.1-3°C/min. After calcination for 4-8 h, a ZSM-5 molecular sieve is obtained, which is H-ZSM-5. In the ZSM-5 molecular sieve, SiO₂/Al₂O₃ is 10-1000.

The molecular sieve catalyst of the present invention may be in H form or in the form of a modified molecular sieve in which H is partially or completely replaced by M, wherein M is selected from one or more of Zn, Ga, Cr, Mn, Fe, Ni, Zr, Cu, La, In and Ca, preferably Zn.

The molecular sieve catalyst in M-modified form can be prepared from the molecular sieve in H form by ion exchanging, impregnation, vapor phase deposition or liquid phase deposition.

When preparing the molecular sieve catalyst in M-modified form by impregnation, the molecular sieve catalyst in H form can be impregnated with an aqueous soluble salt solution of metal M, then dried and calcined, so that M is supported on the molecular sieve catalyst. The soluble salt is, for example, a nitrate salt, a chloride salt, etc.

When preparing the molecular sieve catalyst in M-modified form by ion exchanging, the molecular sieve catalyst in H form can be treated by ion exchanging with an aqueous soluble salt solution of metal M, then dried and calcined.

For example, the process for preparing a Zn-ZSM-5 molecular sieve by ion exchanging is as follows: a H-ZSM-5 molecular sieve is added to an aqueous solution of zinc nitrate, stirred continuously for 10-20 h at 60-100°C to carry out the ion exchanging. After the completion of the ion exchanging, the product is cooled to room temperature, washed until the filtrate has a pH=7-8, dried overnight, and then calcined in a muffle furnace at 400-600°C for 4-6 h to obtain a Zn-ZSM-5 molecular sieve.

In the element M-modified molecular sieve, the element M is 0.1-15 wt% of the total weight of the molecular sieve, preferably 0.5-10 wt%, more preferably 0.7-5 wt%, more preferably 0.7-2 wt%.

The molecular sieve catalyst usually has a particle size of 0.01-20 µm, preferably 0.1-15 µm.

In one embodiment of the present invention, the molecular sieve catalyst can be subjected to a surface modification. When the molecular sieve used is M-modified molecular sieve, the surface modification is carried out after the M modification. The surface modification is carried out with a surface modifying material. Preferably, the surface modifying materials used in the invention are selected from one or more of metal oxides, graphene, activated carbon, Silicalite-1, Silicalite-2, MOF, COF, silica, resin, biomass (such as kelp, glucose, fructose) and carbon nanotubes, more preferably from one or more of activated carbon, Silicalite-1 and Silicalite-2.

The surface modification covers or removes acidic sites exposed on the surface of the molecular sieve catalyst, thus reducing the happening of side reactions, and improving the selectivity toward the target product p-xylene.

The surface modification can be carried out by any conventional methods in the art. As the modification method, hydrothermal synthesis, vapor phase deposition, coating, impregnation, sputtering, Stöber method, and the like may be used, and they can be routinely selected according to the properties of the surface modifying materials. For example, when Silicalite-1 and Silicalite-2 are used for the surface modification, hydrothermal synthesis method can be used. When activated carbon, graphene or carbon nanotubes are used for the modification, vapor phase deposition method can be used. When metal oxides are used for the modification, impregnation and sputtering methods can be used. When silica is used for the modification, Stöber method can be used. When resins or biomasses are used for the modification, coating or impregnation method can be used. Then the obtained surface-modified molecular sieve can be calcined under an inert gas, such as nitrogen, to obtain a carbon surface-modified molecular sieve catalyst.

For example, the process for obtaining H-ZSM-5@Silicalite-1 by modifying H-ZSM-5 with Silicalite-1 by hydrothermal synthesis is as follows: a mixture is prepared from a silicon source (such as TEOS), an organic template (such as TPAOH), ethanol and deionized water, and stirred for 2-6 h at room temperature to obtain a precursor solution of Silicalite-1 molecular sieve. An H-ZSM-5 molecular sieve is transferred into a hydrothermal reactor together with the obtained precursor solution of Silicalite-1 molecular sieve, then sealed, and crystallized for 12-72 h at a rotating speed of 2-5 rmp at a temperature of 100-200°C. After the completion of the crystallization, the obtained product is cooled to room temperature and washed with deionized water until the filtrate has a pH=7-8, dried overnight at room temperature, and then heated in a muffle furnace to 500-650°C at a ramping rate of 0.1-3°C/min. After calcination for 2-8 h, H-ZSM-5@Silicalite-1 is obtained.

In the obtained surface modified molecular sieve catalysts, the weight ratio of the molecular sieve catalyst to the surface modifying material is 100:1-2:1, preferably 50:1-2:1, more preferably 10:1-2:1, especially preferably 5:1-2:1.

In one embodiment of the present invention, the core-shell composite catalyst of the present invention additionally comprises a binder layer between the core and the shell. The binder layer is made of a silicon-containing material, which is preferably selected from silica sol, γ-aminopropyltriethoxysilane (APTES), γ-aminopropyltrimethoxysilane (APTMS), γ-glycidoxypropyl trimethoxysilane, and γ-(methacryloxy)propyl trimethoxysilane, more preferably silica sol.

In the second aspect of the present invention, a method for preparing the core-shell composite catalyst of the present invention is provided, comprising:
1) providing a core in particulate form,
2) providing a molecular sieve catalyst in particulate form, and
3) coating the core with the molecular sieve catalyst.

Before the coating in step 3), the core can be coated with a binder, and then coated with the molecular sieve catalyst. The binder can be a silicon-containing material selected from the group consisting of silica sol, γ-aminopropyltriethoxysilane (APTES), γ-aminopropyltrimethoxysilane (APTMS), γ-glycidoxypropyl trimethoxysilane, and γ-(methacryloxy)propyl trimethoxysilane, preferably silica sol. The core can be impregnated in the binder so that the binder is uniformly applied on the core, and then the core impregnated with the binder is added to the powder of molecular sieve catalyst for coating. The impregnation and coating processes can be repeated for 2-10 times.

After coating, the obtained product is dried and calcined. Drying can be carried out at room temperature to 120°C, preferably at room temperature to 60°C, and most preferably at room temperature. The drying time can be 3-30 hours, preferably 6-20 hours. Calcination can be carried out in a muffle furnace under air atmosphere. The calcination temperature is 350-750°C, preferably 400-600°C, and the calcination time is 1-6 h, preferably 2-4 h.

Surprisingly, it was found that when the core-shell composite catalyst of the present invention is prepared with a binder, the conversion and the selectivity toward p-xylene are significantly improved.

The catalyst prepared by the method of the present invention is a core-shell composite catalyst, which can be used to prepare p-xylene from syngas in one step. The one-step preparation of p-xylene from syngas can be mainly divided into two parts. The first part is the conversion of syngas to methanol, and the other part is the conversion of methanol to p-xylene. The spinel-structure catalyst in the core-shell composite catalyst of the present invention can convert syngas into methanol with high conversion and high selectivity, and the molecular sieve catalyst can further catalyze the methanol converted from syngas into p-xylene. The core-shell composite catalyst of the present invention greatly improves the selectivity toward p-xylene in the xylene products. The inventors found that when using the core-shell composite catalyst of the present invention, both the conversion of carbon monoxide and the selectivity toward p-xylene in the xylene products were significantly improved. In addition, the core-shell composite catalyst of the present invention has advantages of a long service life, simplicity to prepare and easiness to repeat, and has a good application prospect.

In the third aspect of the present invention, use of the core-shell composite catalyst of the present invention as the catalyst in the one-step preparation of p-xylene from syngas is provided.

In the fourth aspect of the present invention, a method for preparing p-xylene from syngas in one step is provided, wherein the core-shell composite catalyst of the present invention or the core-shell composite catalyst prepared by the method of the present invention is used.

Before the composite catalyst of the present invention is used to catalyze the syngas to produce p-xylene, it can be subjected to a reduction pretreatment. Advantageously, the process conditions of the reduction pretreatment are as follows: the reduction gas is pure hydrogen; the pretreatment temperature is 200-800°C, preferably 300-500°C; the pretreatment pressure is 0.1-1.5 MPa, preferably 0.1-0.7 MPa; the volume space velocity of the pretreatment hydrogen is 300-7500 Nm³/h, preferably 600-4500 Nm³/h; and/or the pretreatment reduction time is 2-24 h, preferably 6-8 h. After the reduction pretreatment, syngas is introduced to react to prepare p-xylene. The molar ratio of hydrogen and carbon monoxide in the syngas used for this purpose is 0.1-10, preferably 2-5; the reaction pressure is 1-20 MPa, preferably 2-10 MPa; the reaction temperature is 100-700°C, preferably 350-500°C; and/or the space velocity is 300-7500 Nm³/h, preferably 600-4500 Nm³/h.

When the core-shell composite catalyst of the present invention is used for the conversion of syngas, the conversion of syngas can be above 65%, and the selectivity toward p-xylene in xylene products can be above 80%, which is significantly improved. The core-shell composite catalyst of the present invention can convert syngas into p-xylene in one step without using a multi-stage reactor containing a variety of different types of catalysts, and the reaction process is simpler and easier to operate. In addition, the core-shell composite catalyst of the present invention can maintain high conversion and selectivity within at least 2000 h when used to convert syngas into p-xylene in one step, so it has a long service life.

### Examples

### Example 1

### a. Preparation of a ZnCr₂O₄ catalyst

24.0 g of Cr(NO₃)₃•9H₂O and 8.9 g of Zn(NO₃)₂•6H₂O are dissolved in 90 ml of deionized water. The obtained mixed aqueous nitrate solution is added dropwise into a beaker containing 2 L of deionized water together with an aqueous solution of 1 mol/L (NH₄)₂CO₃ (prepared by dissolving 19.2 g of (NH₄)₂CO₃ in 200 ml of deionized water) for co-precipitation. During the co-precipitation process, the mixture is stirred continuously and incubated at 75°C for 3 h. The pH value is maintained at about 8, which is controlled by the relative flow rate of the two solutions. After the completion of co-precipitation, the mixture is aged at 75°C for 3 h. The precipitates are filtered and washed with deionized water for 3 times. The washed precipitates are dried in an oven at 120°C for 12 h, and then calcined in a muffle furnace at 500°C for 5 h. A methanol synthesis catalyst is obtained, and recorded as a ZnCr₂O₄ catalyst.

### b. Preparation of an H-ZSM-5 molecular sieve

A mixture is prepared by mixing a silicon source (TEOS), an aluminum source (A1(NO₃)₃•9H₂O), an organic template (TPAOH), ethanol and deionized water in a molar ratio (2TEOS: 0.02Al₂O₃: 0.68TPAOH: 8EtOH: 120H₂O), and stirred at room temperature for 6 h to obtain a sol. Then the stirred sol is transferred into a hydrothermal synthesis reactor, sealed, and crystallized for 24 h at a rotating speed of 2 rpm at a temperature of 180°C. After the completion of the crystallization, the product is cooled to room temperature and washed until the filtrate has a pH=7, dried overnight at room temperature, and then heated in a muffle furnace to 550°C at a ramping rate of 1°C/min. After calcination for 6 h, a ZSM-5 molecular sieve is obtained, which is H-ZSM-5. In the ZSM-5 molecular sieve, the Si/A1 molar ratio is 46.

### c. Preparation of a ZnCr₂O₄@ H-ZSM-5 catalyst

The surface of 3 g ZnCr₂O₄ particulate catalyst is impregnated with 10 g of silica sol (ALDRICH, 30 wt.% suspension in H₂O). Subsequently, the excess silica sol is decanted. Then the ZnCr₂O₄ with a wet surface is placed into a round-bottomed flask containing 1 g of powdered H-ZSM-5. The round-bottomed flask is rotated quickly and vigorously to ensure that the surface of ZnCr₂O₄ is completely coated with H-ZSM-5. The coating process is repeated twice, and each time being carried out in a round-bottomed flask containing 1 g of powdered H-ZSM-5. Finally, the catalyst is dried overnight at room temperature and calcined in a muffle furnace at 550°C for 3 h to prepare a ZnCr₂O₄@ H-ZSM-5 catalyst, wherein ZnCr₂O₄ is the core and H-ZSM-5 is the shell, and the mass ratio of the ZnCr₂O₄ catalyst to the H-ZSM-5 molecular sieve is 1:1. It is recorded as a ZnCr₂O₄@ H-ZSM-5 catalyst.

### d. Catalytic experiment

0.5 g of ZnCr₂O₄@ H-ZSM-5 catalyst is filled in a fixed bed high-pressure reactor in the form of a fixed bed, and syngas with a 2.1 molar ratio of H₂ to CO is continuously introduced. The reaction pressure is controlled at 4 MPa; the space velocity of syngas is 1200 Nm³/h; and the reaction temperature is 400°C. After reaction for 4 h, the reaction products and feed gas are analyzed online by gas chromatography, and the reaction results are shown in Table 1.

### Example 2

### a. Preparation of a ZnCr₂O₄ catalyst

The "preparation of a ZnCr₂O₄ catalyst" in Example 1 is repeated to obtain a ZnCr₂O₄ catalyst.

### b. Preparation of an H-ZSM-5 molecular sieve

The "preparation of an H-ZSM-5 molecular sieve" in Example 1 is repeated to obtain an H-ZSM-5 molecular sieve.

### c. Preparation of an H-ZSM-5@ Silicalite-1 catalyst

A mixture is prepared by mixing a silicon source (TEOS), an organic template (TPAOH), ethanol and deionized water in a molar ratio (1.0TEOS: 0.06TPAOH: 16.0EtOH: 240H₂O), and stirred for 4 h at room temperature to obtain a precursor solution of Silicalite-1 molecular sieve. The H-ZSM-5 molecular sieve prepared in step b. is transferred into a polytetrafluoroethylene hydrothermal reactor together with the obtained precursor solution of Silicalite-1 molecular sieve, then sealed, and crystallized for 24 h at a rotating speed of 2 rmp at a temperature of 180°C. After the completion of the crystallization, the obtained product is cooled to room temperature and washed with deionized water until the filtrate has a pH=7, dried overnight at room temperature, and then heated in a muffle furnace to 550°C at a ramping rate of 1 °C/min. After calcination for 4 h, H-ZSM-5@Silicalite-1 is obtained, wherein the weight ratio of the H-ZSM-5 to the Silicalite-1 molecular sieve is 3:1.

### d. Preparation of a ZnCr₂O₄@ H-ZSM-5@ Silicalite-1 catalyst

The "preparation of a ZnCr₂O₄@ H-ZSM-5 catalyst" in Example 1 is repeated, but H-ZSM-5@ Silicalite-1 is used instead of H-ZSM-5 to obtain a ZnCr₂O₄@ H-ZSM-5@ Silicalite-1 catalyst.

### e. Catalytic experiment

The "catalytic experiment" in Example 1 is repeated, but the ZnCr₂O₄@ H-ZSM-5@ Silicalite-1 catalyst is used instead of the ZnCr₂O₄@ H-ZSM-5 catalyst. The reaction results are shown in Table 1.

### Example 3

### a. Preparation of a ZnCr₂O₄ catalyst

The "preparation of a ZnCr₂O₄ catalyst" in Example 1 is repeated to obtain a ZnCr₂O₄ catalyst.

### b. Preparation of an H-ZSM-5 molecular sieve

The "preparation of an H-ZSM-5 molecular sieve" in Example 1 is repeated to obtain an H-ZSM-5 molecular sieve.

### c. Preparation of a Zn-ZSM-5 molecular sieve

1.5 g of H-ZSM-5 molecular sieve is added to an aqueous solution of 1 mol/L of zinc nitrate, and stirred continuously at 80°C for 15 h for ion exchanging. After the completion of the ion exchanging, the obtained product is cooled to room temperature, washed until the filtrate has a pH=7, dried overnight at room temperature, and then calcined in a muffle furnace at 550°C for 5 h to obtain Zn-ZSM-5. Based on the total weight of the Zn-ZSM-5 molecular sieve, the content of Zn is 1%. It is recorded as a Zn-ZSM-5 molecular sieve.

### d. Preparation of a ZnCr₂O₄@Zn-ZSM-5 catalyst

The "preparation of a ZnCr₂O₄@H-ZSM-5 catalyst" in Example 1 is repeated, but Zn-ZSM-5 is used instead of H-ZSM-5 to obtain a ZnCr₂O₄@Zn-ZSM-5 molecular sieve.

### e. Catalytic experiment

The "catalytic experiment" in Example 1 is repeated, but the ZnCr₂O₄@Zn-ZSM-5 catalyst is used instead of the ZnCr₂O₄@ H-ZSM-5 catalyst. The reaction results are shown in Table 1.

### Example 4

### a. Preparation of a ZnCr₂O₄ catalyst

The "preparation of a ZnCr₂O₄ catalyst" in Example 1 is repeated to obtain a ZnCr₂O₄ catalyst.

### b. Preparation of an H-ZSM-5 catalyst

The "preparation of an H-ZSM-5 molecular sieve" in Example 1 is repeated to obtain an H-ZSM-5 molecular sieve.

### c. Preparation of a Zn-ZSM-5 molecular sieve

The "preparation of a Zn-ZSM-5 molecular sieve" in Example 3 is repeated to obtain a Zn-ZSM-5 molecular sieve.

### d. Preparation of a Zn-ZSM-5@ Silicalite-1 catalyst

The "preparation of an H-ZSM-5@ Silicalite-1 catalyst" in Example 2 is repeated, but Zn-ZSM-5 is used instead of H-ZSM-5 to obtain a Zn-ZSM-5@ Silicalite-1 molecular sieve.

### e. Preparation of a ZnCr₂O₄@Zn-ZSM-5@ Silicalite-1 catalyst

The "preparation of a ZnCr₂O₄@H-ZSM-5 catalyst" in Example 1 is repeated, but Zn-ZSM-5@ Silicalite-1 is used instead of H-ZSM-5 to obtain a ZnCr₂O₄@H-ZSM-5@ Silicalite-1 catalyst.

### f. Catalytic experiment

The "catalytic experiment" in Example 1 is repeated, but the ZnCr₂O₄@H-ZSM-5@ Silicalite-1 catalyst is used instead of the ZnCr₂O₄@ H-ZSM-5 catalyst. The reaction results are shown in Table 1.

### Example 5

### a. Preparation of an InGa₂O₄ catalyst

10.6 g of In(NO₃)₃•3H₂O and 23.9 g of Ga(NO₃)₂•8H₂O are dissolved in 90 ml of deionized water. The obtained mixed aqueous nitrate solution is added dropwise into a beaker containing 2 L of deionized water together with an aqueous solution of 1 mol/L (NH₄)₂CO₃ (prepared by dissolving 19.2 g of (NH₄)₂CO₃ in 200 ml of deionized water) for co-precipitation. During the co-precipitation process, the mixture is stirred continuously and incubated at 75°C for 3 h. The pH value is maintained at about 8, which is controlled by the relative flow rate of the two solutions. After the completion of co-precipitation, the mixture is aged at 75°C for 3 h. The precipitates are filtered and washed with deionized water for 3 times. The washed precipitates are dried in an oven at 120°C for 12 h, and then calcined in a muffle furnace at 500°C for 5 h. A methanol synthesis catalyst is obtained, and recorded as an InGa₂O₄ catalyst.

### b. Preparation of an H-ZSM-5 catalyst

The "preparation of an H-ZSM-5 molecular sieve" in Example 1 is repeated to obtain an H-ZSM-5 molecular sieve.

### c. Preparation of a Zn-ZSM-5 catalyst

The "preparation of a Zn-ZSM-5 molecular sieve" in Example 3 is repeated to obtain a Zn-ZSM-5 molecular sieve.

### d. Preparation of a Zn-ZSM-5@ Silicalite-1 catalyst

The "preparation of an H-ZSM-5@ Silicalite-1 catalyst" in Example 2 is repeated, but Zn-ZSM-5 is used instead of H-ZSM-5 to obtain a Zn-ZSM-5@ Silicalite-1 molecular sieve.

### e. Preparation of an InGa₂O₄@Zn-ZSM-5@ Silicalite-1 catalyst

The "preparation of a ZnCr₂O₄@H-ZSM-5 catalyst" in Example 1 is repeated, but Zn-ZSM-5@Silicalite-1 is used instead of H-ZSM-5, and InGa₂O₄ instead of ZnCr₂O₄ to obtain an InGa₂O₄@Zn-ZSM-5@Silicalite-1 catalyst.

### f. Catalytic experiment

The "catalytic experiment" in Example 1 is repeated, but the InGa₂O₄@Zn-ZSM-5@Silicalite-1 catalyst is used instead of the ZnCr₂O₄@ H-ZSM-5 catalyst. The reaction results are shown in Table 1.

### Example 6

### a. Preparation of a ZnCr₂O₄ catalyst

The "preparation of a ZnCr₂O₄ catalyst" in Example 1 is repeated to obtain a ZnCr₂O₄ catalyst.

### b. Preparation of an H-ZSM-11 molecular sieve

A mixture is prepared by mixing a silicon source (TEOS), an aluminum source (Al(NO₃)₃•9H₂O), an organic template (tetrabutylammonium hydroxide (TBAOH)), ethanol and deionized water in a molar ratio (2TEOS: 0.02Al₂O₃: 0.68TPAOH: 8EtOH: 120H₂O), and stirred at room temperature for 6 h to obtain a sol. Then the stirred sol is transferred into a hydrothermal synthesis reactor, sealed, and crystallized for 24 h at a rotating speed of 2 rpm at a temperature of 180°C. After the completion of the crystallization, the obtained product is cooled to room temperature and washed until the filtrate has a pH=7, dried overnight at room temperature, and then heated in a muffle furnace to 550°C at a ramping rate of 1°C/min. After calcination for 6 h, an H-ZSM-11 molecular sieve is obtained, which is H-ZSM-11. In the H-ZSM-11 molecular sieve, the Si/A1molar ratio is 46.

### c. Preparation of a Zn-ZSM-11 catalyst

The "preparation of a Zn-ZSM-5 molecular sieve" in Example 3 is repeated, but H-ZSM-11 is used instead of H-ZSM-5 to obtain a Zn-ZSM-11 molecular sieve.

### d. Preparation of an H-ZSM-11@ Silicalite-1 catalyst

The "preparation of an H-ZSM-5@ Silicalite-1 catalyst" in Example 2 is repeated, but Zn-ZSM-11 is used instead of H-ZSM-5 to obtain an H-ZSM-11@ Silicalite-1 molecular sieve.

### e. Preparation of a ZnCr₂O₄@Zn-ZSM-11@ Silicalite-1 catalyst

The "preparation of a ZnCr₂O₄@H-ZSM-5 catalyst" in Example 1 is repeated, but Zn-ZSM-11@Silicalite-1 is used instead of H-ZSM-5 to obtain a ZnCr₂O₄@H-ZSM-11@Silicalite-1 catalyst.

### f. Catalytic experiment

The "catalytic experiment" in Example 1 is repeated, but the ZnCr₂O₄@H-ZSM-11@Silicalite-1 catalyst is used instead of the ZnCr₂O₄@ H-ZSM-5 catalyst. The reaction results are shown in Table 1.

### Example 7

### a. Preparation of a ZnCr₂O₄ catalyst

The "preparation of a ZnCr₂O₄ catalyst" in Example 1 is repeated to obtain a ZnCr₂O₄ catalyst.

### b. Preparation of an H-ZSM-5 catalyst

The "preparation of an H-ZSM-5 molecular sieve" in Example 1 is repeated to obtain an H-ZSM-5 molecular sieve.

### c. Preparation of a Zn-ZSM-5 catalyst

The "preparation of a Zn-ZSM-5 molecular sieve" in Example 3 is repeated to obtain a Zn-ZSM-5 molecular sieve.

### d. Preparation of a Zn-ZSM-5@ C catalyst

1.5 g of an aqueous solution of glucose with a concentration of 1 mol/L is applied to 1.0 g of Zn-ZSM-5, which is dried overnight at room temperature, then placed in a 600°C tubular furnace, and calcined under nitrogen protection for 5 h to obtain a Zn-ZSM-5@ C catalyst. It is recorded as Zn-ZSM-5@ C.

### e. Preparation of a ZnCr₂O₄@Zn-ZSM-5@ C catalyst

The "preparation of a ZnCr₂O₄@H-ZSM-5 catalyst" in Example 1 is repeated, but Zn-ZSM-5@ C is used instead of H-ZSM-5 to obtain a ZnCr₂O₄@Zn-ZSM-5@ C catalyst.

### f. Catalytic experiment

The "catalytic experiment" in Example 1 is repeated, but the ZnCr₂O₄@Zn-ZSM-5@ C catalyst is used instead of the ZnCr₂O₄@ H-ZSM-5 catalyst. The reaction results are shown in Table 1.

### Comparative Example 1

The catalyst preparation method is essentially the same as that in Example 1, but the core catalyst and the shell catalyst are directly physically mixed to form a physical mixture, and the weight ratio of the core to the shell is changed, so that the weight ratio of the ZnCr₂O₄ catalyst to the H-ZSM-5 molecular sieve in the obtained core-shell composite catalyst is 1:1.

As described in Example 1, the obtained catalyst is applied to the direct preparation of p-xylene from syngas, and the results are shown in Table 1.

### Comparative Example 2

The catalyst preparation method is essentially the same as that in Example 2, but the weight ratio of the H-ZSM-5 to the Silicalite-1 molecular sieve is changed to 1:1.

As described in Example 2, the obtained catalyst is applied to the direct preparation of p-xylene from syngas, and the results are shown in Table 1.

### Comparative Example 3

The catalyst preparation method is essentially the same as that in Example 2, but the weight ratio of the H-ZSM-5 to the Silicalite-1 molecular sieve is changed to 1:3.

As described in Example 2, the obtained catalyst is applied to the direct preparation of p-xylene from syngas, and the results are shown in Table 1.

### Comparative Example 4

The preparation method of catalyst is essentially the same as that in Example 4, but ZnCr₂O₄ and H-ZSM-5@Silicalite-1 are mixed by physical grinding.

As described in Example 4, the obtained catalyst is applied to the direct preparation of p-xylene from syngas, and the results are shown in Table 1.

### Comparative Example 5

The catalyst preparation method is the same as that of Example 2 in "Catalyst for directly preparing p-xylene by using syngas, preparation thereof and applications thereof" (Application Publication Number: CN109590019A).

### Service life test of the catalyst of the present invention

### a. Preparation of a ZnCr₂O₄ catalyst

The "preparation of a ZnCr₂O₄ catalyst" in Example 1 is repeated to obtain a ZnCr₂O₄ catalyst.

### b. Preparation of an H-ZSM-5 molecular sieve

The "preparation of an H-ZSM-5 molecular sieve" in Example 1 is repeated to obtain an H-ZSM-5 molecular sieve.

### c. Preparation of a Zn-ZSM-5 molecular sieve

The "preparation of a Zn-ZSM-5 molecular sieve" in Example 3 is repeated to obtain a Zn-ZSM-5 molecular sieve.

### d. Preparation of a Zn-ZSM-5@ Silicalite-1 catalyst

The "preparation of an H-ZSM-5@ Silicalite-1 catalyst" in Example 2 is repeated, but Zn-ZSM-5 is used instead of H-ZSM-5 to obtain a Zn-ZSM-5@ Silicalite-1 molecular sieve.

### e. Preparation of a ZnCr₂O₄@Zn-ZSM-5@ Silicalite-1 catalyst

The "preparation of a ZnCr₂O₄@H-ZSM-5 catalyst" in Example 1 is repeated, but Zn-ZSM-5@ Silicalite-1 is used instead of H-ZSM-5 to obtain a ZnCr₂O₄@H-ZSM-5@ Silicalite-1 catalyst.

### f. Service life test

0.5 g of ZnCr₂O₄@H-ZSM-5@ Silicalite-1 catalyst is filled in a fixed bed high-pressure reactor in the form of a fixed bed, and syngas with 2.1 molar ratio of H₂ to CO is continuously introduced. The reaction pressure is controlled at 4 MPa; the space velocity of syngas is 1200 Nm³/h; and the reaction temperature is 400°C. The reaction products and feed gas are analyzed online by gas chromatography, and the reaction results are shown in Table 2.

**Table 1**

| Example No. | Conversion (%) | | Selectivity (%) | | | | | | |
|---|---|---|---|---|---|---|---|---|---|
| | CO | MeOH +DME | CH₄ | C₂-C₅ | Others | OX | MX | PX | PX/X |
| Example 1 | 53.2 | 0 | 1.1 | 10.5 | 17 | 2.3 | 10.3 | 58.8 | 82.3 |
| Example 2 | 65.4 | 0 | 1.0 | 11.5 | 0.8 | 2.0 | 9.6 | 75.1 | 86.6 |
| Example 3 | 67.7 | 0 | 1.1 | 13.6 | 8.2 | 2.6 | 11.3 | 63.2 | 81.9 |
| Example 4 | 78.3 | 0 | 0.8 | 3.5 | 3.7 | 0.3 | 1.1 | 90.6 | 98.4 |
| Example 5 | 72.1 | 0 | 1.3 | 6.6 | 1.0 | 1.0 | 5.8 | 84.3 | 92.5 |
| Example 6 | 75.9 | 0 | 1.0 | 8.7 | 2.0 | 0.9 | 6.3 | 81.1 | 91.8 |
| Example 7 | 73.6 | 0 | 1.3 | 5.4 | 9.6 | 3.3 | 4.1 | 76.3 | 91.1 |
| Comparativ e Example 1 | 39.5 | 0 | 3.3 | 19.9 | 11.6 | 2.2 | 8.8 | 54.2 | 83.1 |
| Comparativ e Example 2 | 53.4 | 0 | 2.2 | 36.5 | 0.6 | 4.3 | 7.6 | 48.8 | 80.3 |
| Comparativ e Example 3 | 59.6 | 0 | 0.9 | 17.8 | 2.4 | 3.1 | 12.9 | 62.9 | 79.7 |
| Comparativ e Example 4 | 58.8 | 0 | 2.3 | 23.8 | 10.7 | 2.8 | 13.6 | 46.8 | 74.1 |
| Comparativ e Example 5 | 55.0 | 0 | 4.4 | 33.6 | 26.3 | 0.4 | 0 | 35.3 | 99.0 |

| | | | | | | | | | |
|---|---|---|---|---|---|---|---|---|---|
| Note: MeOH: methanol DME: dimethyl ether C₂-C₅: C₂-C₅ hydrocarbon Others: all other products MX: m-xylene OX: o-xylene PX: p-xylene PX/X: the selectivity toward p-xylene in xylenes | | | | | | | | | |

**Table 2**

| Reactio n time (h) | Conversion (%) | | Selectivity (%) | | | | | | |
|---|---|---|---|---|---|---|---|---|---|
| | CO | MeOH +DME | CH₄ | C₂-C₅ | Others | OX | MX | PX | PX/X |
| 50 | 78.2 | 0 | 0.5 | 3.8 | 3.1 | 0.6 | 0.8 | 91.2 | 98.5 |
| 100 | 78.6 | 0 | 0.4 | 4.1 | 2.6 | 0.7 | 0.7 | 91.5 | 98.5 |
| 200 | 79.1 | 0 | 0.6 | 3.8 | 2.8 | 0.5 | 0.7 | 91.6 | 98.7 |
| 300 | 78.6 | 0 | 0.6 | 3.9 | 2.6 | 0.6 | 0.8 | 91.5 | 98.5 |
| 400 | 78.4 | 0 | 0.5 | 3.7 | 2.8 | 0.7 | 0.6 | 91.7 | 98.6 |
| 500 | 77.9 | 0 | 0.4 | 4.2 | 2.9 | 0.5 | 0.8 | 91.2 | 98.6 |
| 600 | 78.4 | 0 | 0.5 | 4.1 | 3.1 | 0.7 | 0.7 | 90.9 | 98.5 |
| 700 | 79.6 | 0 | 0.5 | 3.6 | 2.5 | 0.6 | 0.7 | 92.1 | 98.6 |
| 800 | 78.3 | 0 | 0.4 | 4.2 | 2.6 | 0.5 | 0.8 | 91.5 | 98.6 |
| 1000 | 79.3 | 0 | 0.6 | 3.9 | 2.7 | 0.7 | 0.6 | 91.5 | 98.6 |
| 1200 | 78.6 | 0 | 0.5 | 3.8 | 2.9 | 0.7 | 0.7 | 91.4 | 98.5 |
| 1400 | 80.6 | 0 | 0.4 | 4.1 | 2.3 | 0.8 | 0.8 | 91.6 | 98.3 |
| 1600 | 77.2 | 0 | 0.6 | 4.0 | 2.8 | 0.6 | 0.7 | 91.3 | 98.6 |
| 1800 | 76.8 | 0 | 0.5 | 3.8 | 3.1 | 0.5 | 0.7 | 91.4 | 98.7 |
| 2000 | 78.5 | 0 | 0.7 | 4.0 | 2.4 | 0.6 | 0.8 | 91.5 | 98.5 |

| | | | | | | | | | |
|---|---|---|---|---|---|---|---|---|---|
| Note: MeOH: methanol DME: dimethyl ether C₂-C₅: C₂-C₅ hydrocarbon Others: all other products MX: m-xylene OX: o-xylene PX: p-xylene PX/X: the selectivity toward p-xylene in xylenes | | | | | | | | | |

## Claims

1. A core-shell composite catalyst, wherein the core is a spinel-structure XYₐO_{b} catalyst, wherein X and Y, being different from each other, are metal elements selected from main group II, transition elements and main group III of the Periodic Table of Elements; a is a number between 1-15, preferably between 1-5; b is the number of oxygen atoms required to satisfy the valence of the elements; the shell is a molecular sieve catalyst, preferably selected from one or more of ZSM-5, ZSM-11, ZSM-35 and MOR, more preferably selected from ZSM-5 and ZSM-11.

2. The core-shell composite catalyst according to claim 1, wherein X and Y are selected from Al, Ga, In, Tl, Zn, Cu, Co, Fe, Mn, Cr, Ti, Mg, Ca and Ba; preferably, X is Ga, In or Zn; and/or Y is Cr, Ga or Ti.

3. The core-shell composite catalyst according to claim 1 or 2, wherein the molecular sieve catalyst is in H form or in the form of a modified molecular sieve in which H is partially or completely replaced by M, wherein M is selected from one or more of Zn, Ga, Cr, Mn, Fe, Ni, Zr, Cu, La, In and Ca, preferably Zn.

4. The core-shell composite catalyst according to any one of claims 1-3, wherein the weight ratio of the spinel-structure XYₐO_{b} catalyst to the molecular sieve catalyst is 150:1-1:50, preferably 20:1-1:20, more preferably 10:1-1:10.

5. The core-shell composite catalyst according to any one of claims 1-4, wherein the molecular sieve catalyst is modified with a surface modifying material selected from one or more of metal oxides, graphene, activated carbon, Silicalite-1, Silicalite-2, MOF, COF, silica, resin, biomass (such as kelp, glucose, fructose) and carbon nanotubes, more preferably from one or more of activated carbon, Silicalite-1 and Silicalite-2.

6. The core-shell composite catalyst according to claim 5, wherein the weight ratio of the molecular sieve catalyst to the surface modifying material in the obtained surface modified molecular sieve catalyst is 100:1-2:1, preferably 50:1-2:1, more preferably 10:1-2:1, especially preferably 5:1-2:1.

7. The core-shell composite catalyst according to any one of claims 1-6, additionally comprising a binder layer between the core and the shell.

8. The core-shell composite catalyst according to claim 7, wherein the binder layer is made of a silicon-containing material preferably selected from silica sol, γ-aminopropyltriethoxysilane (APTES), γ-aminopropyltrimethoxysilane (APTMS), γ-glycidoxypropyl trimethoxysilane, and γ-(methacryloxy)propyl trimethoxysilane, more preferably silica sol.

9. The core-shell composite catalyst according to any one of claims 1-8, wherein the spinel-structure XYₐO_{b} catalyst is ZnCr₂O₄ or InGa₂O₄.

10. A method for preparing the core-shell composite catalyst according to any one of claims 1-9, comprising:
1) providing a core in particulate form,
2) providing a molecular sieve catalyst in particulate form, and
3) coating the core with the molecular sieve catalyst.

11. The method according to claim 10, wherein the core is coated with a binder before being coated with the molecular sieve catalyst.

12. The method according to claim 10 or 11, wherein the binder is a silicon-containing material selected from the group consisting of silica sol, γ-aminopropyltriethoxysilane (APTES), γ-aminopropyltrimethoxysilane (APTMS), γ-glycidoxypropyl trimethoxysilane, and γ-(methacryloxy)propyl trimethoxysilane, preferably silica sol.

13. The method according to any one of claims 10-12, wherein the molecular sieve catalyst is subjected to surface modification with a surface modifying material before being used to coat the core.

14. The method according to any one of claims 10-13, wherein the surface modifying material is selected from one or more of metal oxides, graphene, activated carbon, Silicalite-1, Silicalite-2, MOF, COF, silica, resin, biomass (such as kelp, glucose, fructose) and carbon nanotubes, more preferably from one or more of activated carbon, Silicalite-1 and Silicalite-2.

15. The method according to any one of claims 10-14, wherein the product obtained after coating the core with the molecular sieve catalyst is calcined.

16. Use of the core-shell composite catalyst according to any one of claims 1-9 or the core-shell composite catalyst prepared by the method according to any one of claims 10-15 as the catalyst in the one-step preparation of p-xylene from syngas.

17. A method for preparing p-xylene from syngas in one step, wherein the core-shell composite catalyst according to any one of claims 1-9 or the core-shell composite catalyst prepared by the method according to any one of claims 10-15 is used.

18. The method according to claim 17, wherein the molar ratio of hydrogen to carbon monoxide in the syngas is 0.1-10, preferably 2-5; the reaction pressure is 1-20 MPa, preferably 2-10 MPa; the reaction temperature is 100-700°C, preferably 350-500°C; and/or the space velocity is 300-7500 Nm³/h, preferably 600-4500 Nm³/h.

19. The method according to claim 17 or 18, wherein the catalyst is subjected to a reduction pretreatment before the introduction of syngas for reaction; preferably, the process conditions of the reduction pretreatment are as follows:
the reduction gas is pure hydrogen;
the pretreatment temperature is 200-800°C, preferably 300-500°C;
the pretreatment pressure is 0.1-1.5 MPa, preferably 0.1-0.7 MPa;
the volume space velocity of pretreatment hydrogen is 300-7500 Nm³/h, preferably 600-4500 Nm³/h; and/or
the pretreatment reduction time is 2-24 h, preferably 6-8 h.
